# EUROPÄISCHE PATENTSCHRIFT

(11) **EP 2 049 046 B1**
(45) Veröffentlichungstag und Bekanntmachung des Hinweises auf die Patenterteilung: **21.02.2018**
(21) Anmeldenummer: 07788243.9
(22) Anmeldetag: 06.08.2007
(51) Int. Cl.: A61F 2/34

(54) **ASYMMETRISCHE GESTALTUNG VON HÜFTPFANNEN ZUR VERRINGERUNG DER PFANNENDEFORMATIONEN**
ASYMMETRIC DESIGN OF HIP SOCKET FOR REDUCING SOCKET DEFORMATIONS
CONCEPTION ASYMÉTRIQUE DE COTYLES DE LA HANCHE POUR RÉDUIRE LES DÉFORMATIONS COTYLOÏDES

(30) Priorität: 04.08.2006 DE 102006036924; 06.07.2007 DE 102007031669
(43) Veröffentlichungstag der Anmeldung: 22.04.2009
(73) Patentinhaber: CeramTec GmbH, 73207 Plochingen (DE)
(72) Erfinder: PREUSS, Roman, 70771 Leinf.-Echterdingen (DE); PANDORF, Thomas, 73730 Esslingen-Zell (DE)
(74) Vertreter: Uppena, Franz
(86) Internationale Anmeldenummer: PCT/EP2007/058127
(87) Internationale Veröffentlichungsnummer: WO 2008/015289

(56) Entgegenhaltungen:
- EP-A- 0 380 045
- EP-A- 0 472 315
- EP-A- 0 640 324
- DE-A1- 4 442 559
- DE-A1- 19 701 778
- DE-T2- 69 927 485
- DE-U1- 20 300 018

## Beschreibung

Die Erfindung betrifft eine Hüftpfanne zur Verwendung für eine Hüftgelenkprothese, die aus einem Schaft besteht, auf dem ein Kugelkopf befestigbar ist, der Kugelkopf in einen Pfanneneinsatz drehbar einsetzbar ist, und der Pfanneneinsatz in die Hüftpfanne einsetzbar und fixierbar ist, wobei der Schaft in den Oberschenkelknochen und die Hüftpfanne in den Beckenknochen implantierbar sind.

Hüftpfannen werden im Lauf der Operation zur primären Fixierung durch verschiedene Techniken mit dem Beckenknochen gekoppelt. Eine verbreitete Fixierungsart ist das Klemmen der Pfanne mittels Preßfit. Dies stellt eine Form der Kopplung dar, welche im technischen Sinn den Übermaßverbindungen zuzuordnen ist. D. h. die Außengeometrie der Hüftpfanne ist größer als die vom Arzt durch Ausfräsen des Acetabulum geschaffene Pfannenaufnahme. Die so nach dem Einbringen der Pfanne durch den Knochen auf die Pfanne wirkenden Normalkräfte und infolgedessen Reibkräfte stellen die primäre Verankerung der Hüftpfanne sicher.

Aufgrund der inhomogenen Steifigkeit des Beckenknochens erfolgt in der Regel eine asymmetrische Belastung der Hüftpfanne in der Preßfit-Situation, welche eine asymmetrische Verformung der Hüftpfanne zur Folge haben kann. Dies ist prinzipiell unerwünscht, da die Verformung der Hüftpfanne das Einbringen der Pfanneneinsätze erschwert bzw. weiterhin zur Belastung und asymmetrischen Verformung der Pfanneneinsätze führen kann. Mögliche weitere Auswirkungen sind dann Beschädigung des Pfanneneinsatzes und/oder Einschränkung der Funktion der Gleitpaarung durch lokale Verringerung des Kalottendurchmessers des Pfanneneinsatzes.

Die Inhomogenität des Beckenknochens bzgl. Steifigkeit liegt in der Regel in der immer gleichen Richtung vor, so dass der Arzt die Richtung der höchsten Belastung für die Hüftpfanne im Preßfit mit hinreichender Genauigkeit am Patienten abschätzen kann.

Die Merkmale des Oberbegriffs des Anspruchs 1 sind aus DE 69927485 T2 bekannt. Der Erfindung liegt die Aufgabe zugrunde, eine Hüftpfanne nach dem Oberbe-griff des Anspruchs 1 derart weiterzubilden, dass eine Beschädigung des Pfanneneinsatzes und/oder eine Einschränkung der Funktion der Gleitpaarung Kugelkopf/Pfanneneinsatz durch lokale Verringerung des Kalottendurchmessers des Pfanneneinsatzes vermieden sind.

Erfindungsgemäß wird diese Aufgabe durch die Merkmale des Anspruchs 1 gelöst. Durch die gezielte asymmetrische Gestaltung der Hüftpfanne bzgl. ihrer Steifigkeit kann der asymmetrischen äußeren Belastung in der Preßfit-Situation entgegengewirkt werden. Die Hüftpfanne weist somit in zwei orthogonalen Richtungen unterschiedliche Steifigkeiten auf.

Die Achse höherer Steifigkeit der Hüftpfanne kann bei der Implantation kolinear zur Richtung der höchsten Steifigkeit des Beckenknochens ausgerichtet werden. Durch entsprechende Auslegung der Steifigkeiten der Hüftpfanne in Relation zu den Steifigkeiten des Beckenknochens kann erreicht werden, dass die unvermeidliche Verformung der Hüftpfanne durch die Preßfit-Situation gleichmäßig auftritt, d. h. mit nahezu betragsgleichen Verformungswegen auf dem gesamten Pfannenumfang.

In einer erfinderischen Ausgestaltung weist die Hüftpfanne in einer ersten Richtung eine minimale Wandstärke und in einer zweiten Richtung eine maximale Wandstärke auf. Diese asymmetrische Gestaltung der Wandstärke der Hüftpfanne wird zum Beispiel so erreicht, dass im Bereich der Stirnfläche in einer Richtung eine minimale Wandstärke und orthogonal dazu eine maximale Wandstärke realisiert ist. Aufgrund der unterschiedlichen Wandstärken, weist die Hüftpfanne in den beiden genannten Richtungen ebenfalls unterschiedliche Steifigkeit auf. Erfindungsgemäss sind in einer Richtung in Bezug auf die andere Richtung versteifende Elemente in die Hüftpfanne eingebracht. Durch Einbringen von versteifenden Elementen - Elemente aus einem Material mit höherer Steifigkeit - in den Mantel der Hüftpfanne können ebenfalls unterschiedliche Steifigkeiten der Hüftpfanne bei verschiedenen Belastungsrichtungen erreicht werden. Die versteifenden Elemente einer Richtung verringern ihre Steifigkeit zur anderen Richtung hin stetig. In einer weiteren Ausführungsform ist die Geometrie der Hüftpfanne derart asymmetrisch ausgebildet, dass sich bei asymmetrischer Belastung eine möglichst kreisringförmige symmetrische Geometrie ergibt. Vorteilhaft ist der Querschnitt der Hüftpfanne senkrecht zur Symmetrieachse im unbelasteten Zustand oval.

Durch asymmetrische Gestaltung der Pfannengeometrie, z.B. des belasteten Pfannenquerschnitts kann erreicht werden, dass bei Vorliegen einer asymmetrischen Belastung eine ebenfalls asymmetrische Verformung auftritt, welche zur Ausbildung einer symmetrischen Pfannengeometrie führt. Im konkreten Beispiel wird ein ovaler Pfannenquerschnitt infolge der asymmetrischen Belastung im Preßfit verformt, bis der Querschnitt eine nahezu kreisringförmige Geometrie aufweist.

Die Hüftpfanne ist bevorzugt aus zumindest einem Metall gefertigt.

Nachfolgend werden der Stand der Technik und die Erfindung anhand von Figuren näher erläutert.

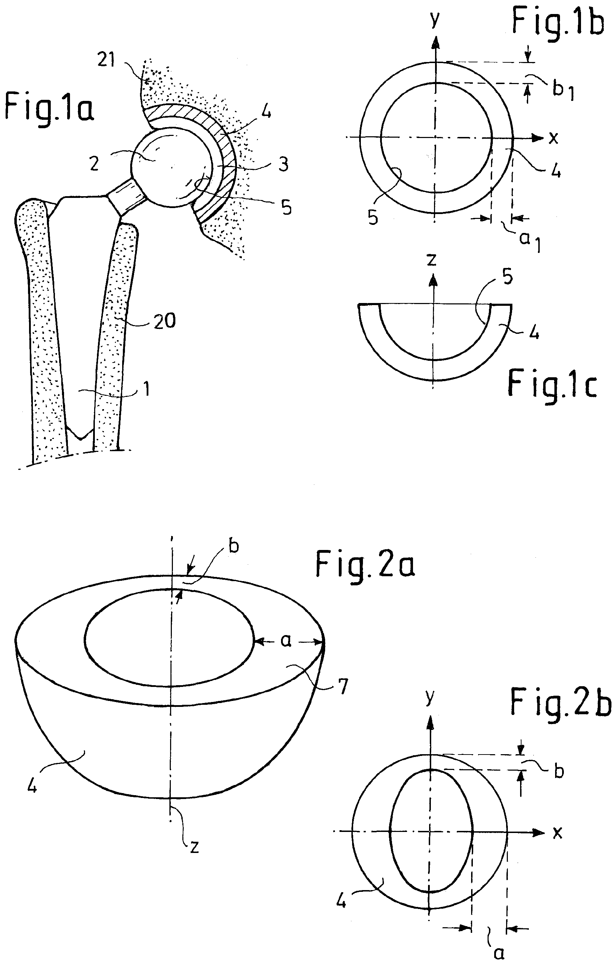

Die Figuren 1a, 1b, 1c zeigen den Stand der Technik. Eine Hüftgelenkprothese besteht in der Regel aus einem Schaft 1 gekoppelt mit einem Kugelkopf 2 und einer Hüftpfanne 4 gekoppelt mit einem Pfanneneinsatz 3. Schaft 1 und Hüftpfanne 4 sind mit dem Körper des Patienten durch Einwachsen in den Oberschenkel- 20 bzw. Beckenknochen 21 verbunden und sind Träger für den Kugelkopf 2 bzw. Pfanneneinsatz 3. Der Kugelkopf 2 ist in der Kalotte 5 des Pfanneneinsatzes 3 drehbar gelagert. Die Hüftpfanne 4 ist bezüglich ihrer Symmetrieachse z rotationssymmetrisch ausgebildet, wodurch die Wandstärken a₁, b₂ der Hüftpfanne 4 in allen Richtungen x, y gleich sind und eine asymmetrische Belastung der Hüftpfanne 4 in der Preßfit-Situation eine asymmetrische Verformung der Hüftpfanne 4 zur Folge haben kann.

Figur 2a, b zeigt eine Hüftpfanne 4, die in der Ebene der Stirnfläche 7 nicht symmetrisch (siehe Figur 2a) aufgebaut ist. Die Wandstärke a in einer zur Symmetrieachse z orthogonalen Richtung ist maximal und die Wandstärke b ebenfalls in einer ebenfalls zur Symmetrieachse z orthogonalen Richtung y ist minimal. Die Richtungen x, y sind dabei orthogonal, d.h. rechtwinklig zueinander angeordnet.

Aufgrund der unterschiedlichen Wandstärken a, b weist die Hüftpfanne 4 in den beiden genannten Richtungen x, y unterschiedliche Steifigkeiten auf.

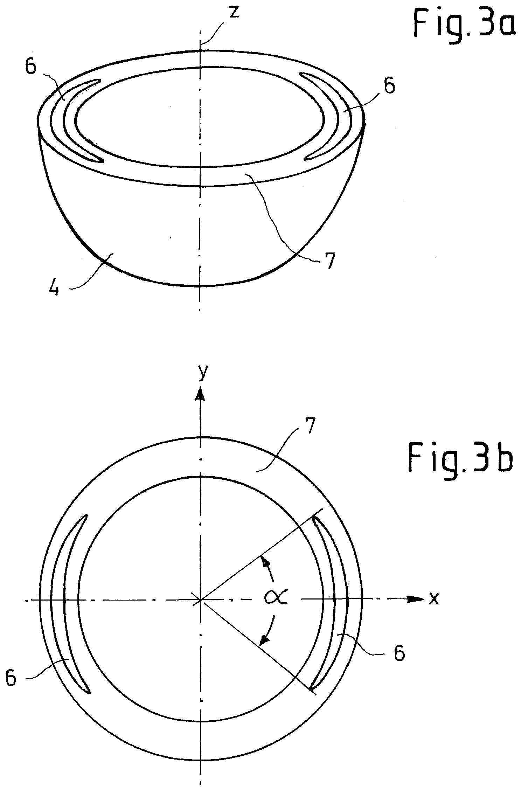

Figur 3 zeigt eine erfindungsgemäße Hüftpfanne 4, bei der in einer Richtung x in Bezug auf die andere orthogonale Richtung y versteifend wirkende Elemente 6 in die Hüftpfanne eingebracht sind. Durch Einbringen dieser versteifenden Elemente 6 - Elemente aus einem Material mit höherer Steifigkeit als das restliche Material der Hüftpfanne 4 - in den Mantel der Hüftpfanne 4 sind unterschiedliche Steifigkeiten der Hüftpfanne 4 bzgl. verschiedener Belastungsrichtungen realisiert.

Die versteifenden Elemente 6 einer Richtung x umfassen dabei einen Winkelbereich α auf der Stirnfläche von annähernd 90 Grad (siehe Figur 3 b). Wichtig ist, dass sich die Steifigkeit in einer Richtung x von der Steifigkeit in einer zur Richtung x orthogonalen Richtung y unterscheidet. Um eine homogene Steifigkeitsänderung zwischen den beiden Extremwerten bzgl. der Richtung x und y zu erreichen, können die versteifenden Elemente einen Winkel α von bis zu 180° umfassen, wobei die Wandstärke des jeweiligen Elements von einem Ende bis zur Mitte des Elements kontinuierlich zu und zum anderen Ende hin genauso kontinuierlich wieder abnimmt.

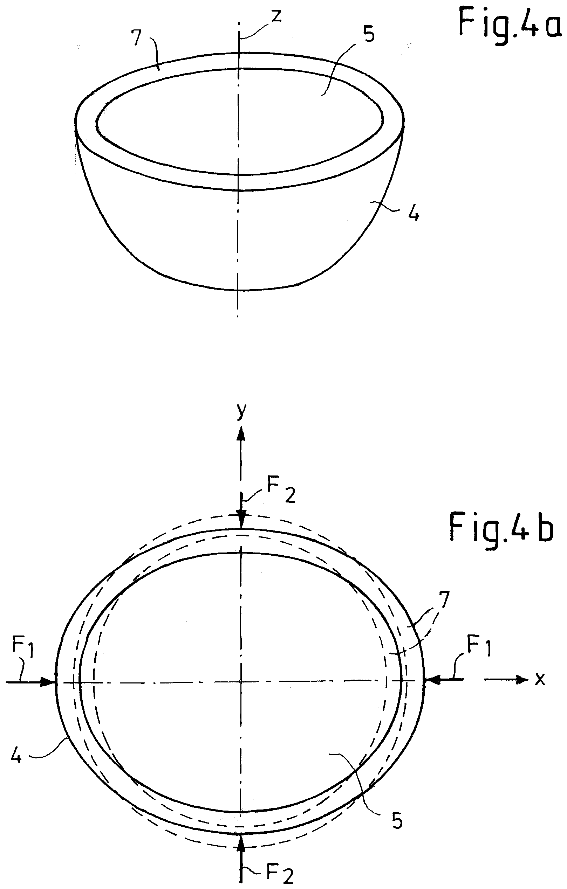

Figur 4b zeigt eine Hüftpfanne 4, deren Geometrie asymmetrisch derart ausgebildet ist, dass der Querschnitt der Hüftpfanne 4 senkrecht zur Symmetrieachse z im unbelasteten Zustand (durchgezogene Linie) oval ist. Bei einer asymmetrischen Belastung verformt sich die Hüftpfanne 4 (siehe gestrichelte Linie in Figur 4 b), so dass als Resultat eine symmetrische Geometrie auftritt. Figur 4 a zeigt die Hüftpfanne 4 im belasteten Zustand. Die in vereinfachter Modellvorstellung punktuell auftretenden Kräfte sind mit F₁, F₂ gekennzeichnet. Es ist bei dieser Ausführungsform sicherzustellen, dass sich bei asymmetrischer Belastung (in Richtung F₁) eine möglichst kreisringförmige symmetrische Geometrie (siehe Figur 4 a) ergibt.

Durch diese asymmetrische Gestaltung der Geometrie der Hüftpfanne 4 kann bei einer asymmetrischen Belastung eine ebenfalls asymmetrische Verformung auftreten, welche zur Ausbildung einer symmetrischen Pfannengeometrie führen kann. Im konkreten Beispiel wird ein ovaler Pfannenquerschnitt infolge der asymmetrischen Belastung im Preßfit verformt, bis der Querschnitt eine nahezu kreisringförmige Geometrie aufweist.

## Patentansprüche

1. Hüftpfanne zur Verwendung für eine Hüftgelenkprothese, die aus einem Schaft (1) besteht, auf dem ein Kugelkopf (2) befestigbar ist, der Kugelkopf (2) in einen Pfanneneinsatz (3) drehbar einsetzbar ist, und der Pfanneneinsatz (3) in die Hüftpfanne (4) einsetzbar und fixierbar ist, wobei der Schaft (1) in den Oberschenkelknochen (20) und die Hüftpfanne (4) in den Beckenknochen (21) implantierbar sind, und wobei die Hüftpfanne (4) bezüglich ihrer Steifigkeit in zwei zueinander und zur Symmetrieachse (z) der Hüftpfanne (4) orthogonalen Richtungen (x, y) asymmetrisch ausgebildet ist, **dadurch gekennzeichnet, dass** in einer Richtung (x) in Bezug auf die andere Richtung (y) versteifende und einander gegenüberliegende Elemente (6) in den Mantel der Hüftpfanne (4) eingebracht sind, wobei die versteifenden Elemente (6) einer Richtung (x) ihre Steifigkeit zur anderen Richtung (y) stetig verringern.

2. Hüftpfanne nach Anspruch 1, **dadurch gekennzeichnet, dass** die Hüftpfanne (4) in einer ersten Richtung (y) eine minimale Wandstärke (b) und in einer zweiten Richtung (x) eine maximale Wandstärke (a) aufweist.

3. Hüftpfanne nach Anspruch 1 oder 2, **dadurch gekennzeichnet, dass** die Geometrie der Hüftpfanne (4) derart asymmetrisch ausgebildet ist, dass sich bei asymmetrischer Belastung eine möglichst kreisringförmige symmetrische Geometrie ergibt.

4. Hüftpfanne nach Anspruch 3, **dadurch gekennzeichnet, dass** der Querschnitt der Hüftpfanne (4) senkrecht zur Längsachse (z) im unbelasteten Zustand oval ist.

5. Hüftpfanne nach einem der Ansprüche 1 bis 4, **dadurch gekennzeichnet, dass** die Hüftpfanne (4) aus zumindest einem Metall gefertigt ist.

## Claims

1. A hip socket for use for a hip-joint prosthesis that consists of a shaft (1) on which a ball head (2) can be secured, the ball head (2) can be rotatably inserted into a socket insert (3), and the socket insert (3) can be inserted into the hip socket (4) and fixed, wherein the shaft (1) can be implanted in the
femur (20), and the hip socket (4) can be implanted in the pelvic bone (21), and
wherein the hip socket (4) is formed asymmetrically with regard to its rigidity in two directions (x, y) that are orthogonal to each other and to the axis of symmetry (z) of the hip socket (4),
**characterised in that** elements (6) that are located opposite each other and act in a stiffening manner in one direction (x) in relation to the other direction (y) are introduced into the casing of the hip socket (4), wherein the stiffening elements (6) of one direction (x) constantly reduce the rigidity thereof with respect to the other direction (y).

2. A hip socket according to claim 1, **characterised in that** the hip socket (4) has a minimum wall thickness (b) in a first direction (y) and a maximum wall thickness (a) in a second direction (x).

3. A hip socket according to claim 1 or 2, **characterised in that** the geometry of the hip socket (4) is formed asymmetrically in such a way that in the event of asymmetrical loading a symmetrical geometry results that is as circular-ring-shaped as possible.

4. A hip socket according to claim 3, **characterised in that** the cross-section of the hip socket (4) is oval perpendicularly to the longitudinal axis (z) in the unloaded state.

5. A hip socket according to one of claims 1 to 4, **characterised in that** the hip socket (4) is made from at least one metal.

## Revendications

1. Cotyle de la hanche destiné à l'utilisation pour une prothèse d'articulation de la hanche, qui se compose d'une tige (1) sur laquelle peut être fixée une rotule (2), la rotule (2) pouvant être insérée de manière rotative dans un insert de cupule (3) et l'insert de cupule (3) pouvant être inséré et fixé dans le cotyle (4), la tige (1) pouvant être implantée dans l'os du fémur (20) et le cotyle (4) pouvant être implanté dans l'os du bassin (21) et le cotyle (4) étant réalisé de manière asymétrique en termes de rigidité dans deux directions (x, y) perpendiculaires l'une à l'autre et à l'axe de symétrie (z) du cotyle (4), **caractérisé en ce que** des éléments (6) de renforcement dans une direction (x) par rapport à l'autre direction (y) et opposés l'un à l'autre sont introduits dans l'enveloppe du cotyle (4), les éléments (6) de renforcement dans une direction (x) réduisant constamment leur rigidité par rapport à l'autre direction (y).

2. Cotyle selon la revendication 1, **caractérisé en ce que** le cotyle (4) présente dans une première direction (y) une épaisseur de paroi minimale (b) et dans une deuxième direction (x) une épaisseur de paroi maximale (a).

3. Cotyle selon la revendication 1 ou 2, **caractérisé en ce que** la géométrie du cotyle (4) est réalisée de manière asymétrique de telle sorte que lors d'une sollicitation asymétrique, on obtienne une géométrie symétrique de forme annulaire circulaire dans la mesure du possible.

4. Cotyle selon la revendication 3, **caractérisé en ce que** la section transversale du cotyle (4) est ovale dans l'état non sollicité perpendiculairement à l'axe longitudinal (z).

5. Cotyle selon l'une quelconque des revendications 1 à 4, **caractérisé en ce que** le cotyle (4) est fabriqué à partir d'au moins un métal.
